(19) **Europäisches Patentamt / European Patent Office / Office européen des brevets**

(11) **EP 1 922 548 B1**

(12) **EUROPEAN PATENT SPECIFICATION**

(45) Date of publication and mention of the grant of the patent:
**16.05.2012 Bulletin 2012/20**

(51) Int Cl.:
***G01N 33/72*** *(2006.01)*

(21) Application number: **06802150.0**

(22) Date of filing: **23.08.2006**

(86) International application number:
**PCT/US2006/032897**

(87) International publication number:
**WO 2007/030325 (15.03.2007 Gazette 2007/11)**

(54) **METHOD OF HEMOGLOBIN CORRECTION DUE TO TEMPERATURE VARIATION**

VERFAHREN ZUR HÄMOGLOBINKORREKTUR AUFGRUND VON TEMPERATURVARIATIONEN

PROCEDE DE CORRECTION D'HEMOGLOBINE DUE A UNE VARIATION DE TEMPERATURE

(84) Designated Contracting States:
**DE FR GB**

(30) Priority: **08.09.2005 US 222457**

(43) Date of publication of application:
**21.05.2008 Bulletin 2008/21**

(73) Proprietor: **Beckman Coulter, Inc.
Brea, CA 92821 (US)**

(72) Inventors:
• **HUO, Ziling
Miami, FL 33176 (US)**
• **LI, William, W.
Miami, FL 33157 (US)**

• **QIAN, Cheng
Miami, FL 33196 (US)**

(74) Representative: **Draper, Martyn John et al
Boult Wade Tennant
Verulam Gardens
70 Gray's Inn Road
London WC1X 8BT (GB)**

(56) References cited:
**EP-A1- 0 210 417    WO-A2-2005/054811
GB-A- 2 077 927    US-A- 3 549 994
US-A- 4 013 417    US-A- 4 134 678
US-A- 5 968 832**

**Description**

This invention relates to the field of hematology, and more particularly to the field of automated hematology analyzers.

It is common medical diagnostic practice to obtain a sample of a patient's blood and test the sample for various hematology parameters. For example, a patient's blood sample may be tested and analyzed to determine red blood cell count, platelet count, white blood cell count, white blood cell types, hematocrit and/or hemoglobin concentration. A number of other hematology parameters may also be determined and analyzed.

The parameters of the patient's blood revealed by the blood testing and analysis may be of significant assistance to a physician in making a diagnosis. For example, increased white blood cell count may indicate the existence of an infection in the body. Certain increased concentrations of white blood cells may indicate various conditions, such as leukemia. A high red blood cell count may indicate that the patient is not receiving enough oxygen and may suggest a condition such as lung disease or heart disease. A low red blood cell count may indicate that the patient is anemic.

Hemoglobin is the major substance in red blood cells. It carries oxygen and gives the blood its red color. Hemoglobin information is one parameter that the physician may use in making a diagnosis. For example, the amount of hemoglobin in the blood is a good indicator of the blood's ability to cany oxygen throughout a patient's body. A high hemoglobin value may be caused by a number of factors such as lung disease, heart disease or kidney disease. A low hemoglobin value may indicate anemia. Hemoglobin parameters may also be valuable in determining a patient's responsiveness to certain therapies, such as therapies directed toward diseases which affect hemoglobin. In addition to analyzing hemoglobin values, analysis may also be conducted on the various types of hemoglobin in the body. While there are only three types of normal hemoglobin, more than three hundred abnormal hemoglobin types have been discovered in patients with certain clinical symptoms. Abnormal hemoglobin types are often indicative of various conditions and/or diseases.

Automated hematology analyzers are currently used for measuring various hematology parameters of a patient's blood, including hemoglobin parameters such as hemoglobin concentration. These automated hematology analyzers are operable to analyze a number of hematology parameters, including white blood cell count, red blood cell count, platelet count and hemoglobin concentration.

When measuring hemoglobin concentration, the automated hematology analyzer takes a blood sample and first dilutes the sample with a diluent. A hemolytic reagent is then added to the diluted sample in order to lyse the red blood cells in the sample. Lysing the diluted sample converts the hemoglobin in the sample to methemoglobin. The methemoglobin is then complexed to form a relatively stable chromogen which is able to be detected and measured by UV spectroscopy at a given wavelength.

Following production of the chromogen in the lysed test sample, the test sample is passed through a hemoglobin absorption cuvette. A light source oriented on one side of the cuvette emits light through the cuvette. The light source emits light at a frequency at or near the peak absorption of the chromogen in the diluted sample (e.g., 540 nm). A detector positioned on the opposite side of the cuvette is used to detect the light that passes through the cuvette and sample. The detector and light may be provided as part of a spectrophotometer or other instrument operable to determine the absorption (or transmittance) of the light through the cuvette and sample. The absorption measurement obtained by the detector is then translated into a corresponding hemoglobin concentration for the sample. This translated hemoglobin concentration is multiplied by a calibration factor for the automated hematology analyzer to arrive at a final hemoglobin concentration measurement for the sample.

It has been noted that the temperature at which a hemoglobin measurement is taken for a blood sample has an effect on the hemoglobin measurement for such blood sample. One important reason for this is that the chromogen produced by the reaction of the diluted sample with the hemolytic reagent is not sufficiently stable to avoid sensitivity to its environment. The result is that the absorption of the chromogen varies with temperature. Because the absorption of the chromogen varies with temperature, different hemoglobin measurements may be obtained from a single sample depending upon the temperature of the sample when the measurement is taken. However, it should be noted that hemoglobin concentration is not the only hematology parameter that varies with temperature, as cellular size, counts and sub-population distribution may vary with temperature along with other hematology parameters.

In addition to variation with temperature, hemoglobin concentration and other hematology parameters may vary with time. In the case of hemoglobin concentration, the absorption of the chromogen produced from the hemolytic reaction decays with time. Accordingly, when obtaining a hematology measurement such as hemoglobin concentration, it is generally not acceptable to wait for the lysed sample to reach a steady state temperature. Instead, the absorption measurement must be taken relatively quickly following the reaction of the diluted sample with the hemolytic reagent. Since the measurement must be taken relatively quickly, some attempt must be made to deal with the temperature fluctuation of the lysed and diluted sample if an accurate hemoglobin measurement is desired.

Unfortunately, it is not easy to produce chromogen from the hemolytic reaction at a single stable temperature immediately following the reaction. For example, hemolytic reagents used to lyse hemoglobin often result in different reaction temperatures, and these reaction temperatures vary over time. Additionally, the environmental temperature of a laboratory may have an effect on reaction temperature.

Several prior art systems and methods have been proposed and used in an attempt to avoid fluctuating hemoglobin measurements because of temperature variations. However, these prior systems and methods have not produced satisfactory results, as significant temperature variations continue to produce different hemoglobin measurements when using these systems methods.

One proposed method for reducing the affects of temperature in hemoglobin measurements involves selecting ligands for the hemolytic reagent with high affinity to provide more stable chromogens that do not significantly vary with temperature, such as that disclosed in U.S. Patent No. 5,763,280. Another method for reducing the effects of temperature on hemoglobin measurement involves using a hemoglobin stabilizer, such as that disclosed in U.S. Patent No. 5,968,832. However both of these methods are unsatisfactory in their results as well as their additional costs.

The calibration method is another example of a prior art method for reducing the effects of temperature variation on hemoglobin measurement. The calibration method is used by many current automated hematology analyzers (see WO 2005/054811 for example). This method acknowledges that the initial uncalibrated hemoglobin measurement taken by the automated hematology analyzer is not always accurate because of various factors such as engineering tolerances and environmental factors, and unique instrument characteristics. Using this method, an initial uncalibrated hemoglobin measurement is first obtained using the automated hematology analyzer. This uncalibrated measurement is then multiplied by a calibration factor to arrive at the calibrated hemoglobin measurement (e.g., $Hgb_{Final}$ = CalibrationFactor * $Hgb_{Uncalibrated}$). The calibration factor is generally determined by empirical testing and programmed into the instrument before it is sold. The same calibration factor is applied to all hemoglobin measurements made with the instrument or to hemoglobin measurements made within a certain temperature operating range. While the calibration method provides for scaling of the measured temperature, these same changes are generally applied to all measurements or a whole range of measurements, and are not exact changes that account for temperature variations over a range of temperatures. Accordingly, the temperature calibration method provides generally unsatisfactory results when attempting to accurately measure hemoglobin.

Yet another example of a prior art method for reducing the effects of temperature variation on hemoglobin measurement is the temperature control method (see GB 2077927 for example). The temperature control method involves the use of an automated hematology analyzer having a built-in temperature control unit. The temperature control unit in such an automated hematology analyzer generally warms the hemoglobin reaction temperature to a predetermined temperature such that all hemoglobin measurements using the automated hematology analyzer are taken at nearly the same temperature. Unfortunately, inclusion of a temperature control unit within the automated hematology has several problems. For example, the inclusion of the temperature control unit adds significant cost to the instrument which is then passed on to the purchaser of the instrument in the form of an increased price. Furthermore, the temperature control unit adds size to the instrument, and space is often a valuable resource in the laboratory environment. In addition, when a temperature control unit is added, additional parts are included in the machine that make the machine more susceptible to failure and need of repair. Moreover, even with a temperature control unit, measurement results are not always accurate, as the hemoglobin reaction temperature may change frequently or may be higher than expected (e.g., higher than the predetermined temperature), resulting in a measurement being taken before the temperature control unit stabilizes the temperature to the predetermined temperature.

Accordingly, it would be desirable to provide an automated hematology analyzer that is capable of accurately measuring various hematology parameters of a sample, such as hemoglobin concentration, at various sample temperatures, and does not require a temperature control unit.

According to a first aspect of the invention, there is provided a method of measuring a hematology parameter of a test sample, the method comprising measuring a temperature corresponding to the test sample; obtaining a first measurement of the hematology parameter of the test sample; and determining a corrected measurement of the hematology parameter of the sample at the measured temperature, wherein the corrected measurement is an empirical function of the measured temperature, a reference temperature, and the first measurement of the hematology parameter, wherein the empirical function is regressed using measurements of hemoglobin variation at different temperatures taken from multiple patients.

According to a second aspect of the invention, there is provided an automated hematology analyzer for measuring a haemoglobin parameter of a test sample, the automated hematology analyzer comprising a cuvette operable to receive a test sample; a light source operable to emit light toward the cuvette; a detector operable to detect the amount of light from the light source passing through the cuvette and provide a first measurement of the haemoglobin parameter; a temperature sensor operable to measure a temperature corresponding to the test sample; and a processor operable to determine a corrected measurement of the hemoglobin parameter, wherein the corrected measurement is an empirical function of the first measurement, a reference temperature and the operating temperature, wherein the empirical function is regressed using measurements of hemoglobin variation at different temperatures taken from multiple patients.

According to a third aspect of the invention, there is provided a method of measuring a haemoglobin parameter of a sample temperature, the method comprising providing a test sample to an automated hematology analyzer; diluting and lysing the test sample; obtaining a temperature measurement corresponding to the diluted and lysed test sample; obtaining a first measurement of the haemoglobin parameter of the test sample at the measured temperature; and determining a corrected measurement of the hemoglobin parameter, wherein the corrected measurement is an empirical function of the first hemoglobin measurement, a reference temperature and the measured temperature, wherein the empirical function is regressed using measurements of hemoglobin variation at different temperatures taken from multiple patients.

According to a fourth aspect of the invention, there is provided an automated hematology analyzer for measuring a hemoglobin parameter of a sample at an operating temperature, the automated hematology analyzer comprising means for obtaining a first measurement of the haemoglobin parameter of the sample at the operating temperature; means for measuring the operating temperature; and means for calculating a corrected measurement of the haemoglobin parameter at the operating temperature, wherein the corrected measurement is an empirical function of the first haemoglobin measurement, a reference temperature and the operating temperature, wherein the empirical function is regressed using measurements of hemoglobin variation at different temperatures taken from multiple patients.

In the accompanying drawings:

FIG. 1 shows a schematic view of an automated hematology analyzer;

FIG. 2 shows a block diagram of a series of steps taken to provide a corrected haemoglobin measurement for the automated hematology analyzer of FIG. 1;

FIG. 3A shows a graph of uncorrected haemoglobin concentration results for a group of blood samples; and

FIG. 3B shows a graph of corrected haemoglobin concentration results from the blood samples of FIG. 3A using the method described herein.

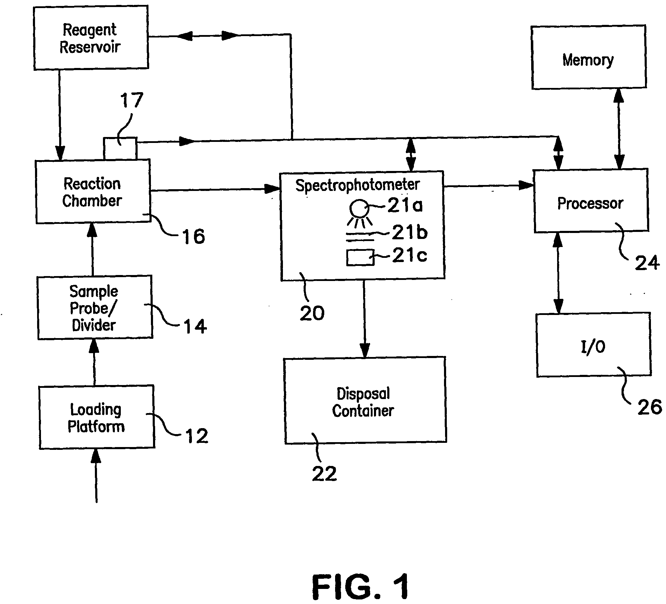

With reference to FIG. 1, an automated hematology analyzer 10 is shown in block diagram form. The automated haemoglobin analyzer 10 is operable to provide a corrected haemoglobin measurement due to temperature variation. The analyzer 10 includes a loading platform 12, a sample divider 14, a reaction chamber 16, temperature sensor 17, reagent reservoir 18, spectrophotometer 20, disposal container 22, processor 24, and I/O device.

The loading platform 12 is designed to receive a test vial/test tube containing a test sample of a patient's blood. The loading platform takes the vial and delivers its contents to the sample divider 14. The sample divider splits the sample into at least a first aliquot and a second aliquot. The first aliquot is delivered to the reaction chamber 16 in one portion of the automated hematology analyzer operable to determine white blood cell count and hemoglobin concentration. The second aliquot is delivered to another portion (not shown) of the automated hematology analyzer operable to determine red blood cell count and platelet count. In FIG. 1, the portion of the automated hematology analyzer that processes the first aliquot and obtains a hemoglobin concentration is represented.

After the blood sample is divided into separate aliquots, the first aliquot is delivered to a reaction chamber 16. The reaction chamber is generally a relatively large chamber compared to the size of the first aliquot. For example, in one embodiment, aliquots of about 28 $\mu$l are delivered to a reaction chamber that is between 7,000 $\mu$l and 10,000 $\mu$l in size. The reaction chamber is relatively large, because a large amount of diluent and/or hemolytic reagent is added to the test sample in the reaction chamber 16. For example, a 28ml test sample may be combined with about 6,000 $\mu$l of diluent and about 1,000 $\mu$l of lysing agent in the reaction chamber 16. A reagent reservoir 18 is connected to the reaction chamber 16 and is operable to deliver the diluent and/or hemolytic reagent to the reaction chamber 16 when needed.

A temperature sensor 17 is connected to the reaction chamber and is operable to take a temperature of the reaction chamber. In one embodiment, the temperature sensor is a thermistor. However, the temperature sensor may take the form of any number of other types of temperature sensors operable to provide accurate and precise temperature readings, such as resistance temperature devices (RTDs), thermometers, IR thermometers, and thermocouples. The temperature taken by the temperature sensor 17 corresponds to the test sample in some manner. In the embodiment of FIG. 1, the temperature taken by the temperature sensor 17 corresponds to the test sample, because the temperature of the reaction chamber 16 is a good indication of the temperature of the diluted and lysed test sample before it is delivered to the spectrophotometer. However, the temperature taken by the temperature sensor may be taken in other locations and still correspond to the temperature of the test sample. In one embodiment, the temperature sensor measures the temperature of the diluent before it is added to the reaction chamber. In this embodiment, the temperature of the diluent corresponds to the temperature of the test sample since the volume of diluent is significantly greater than the volume of the test sample it is mixed with. In another embodiment, the temperature sensor measures the ambient air temperature of the laboratory where the automated hematology analyzer is positioned is measured. In this embodiment, the ambient air temperature corresponds to the temperature of the test sample, with at least the diluent and possibly the test sample at or near the ambient air temperature. In yet another embodiment, the temperature sensor measures the temperature of the cuvette of the photocolorimeter, as the temperature of the cuvette corresponds to the temperature of the diluted and lysed test sample.

The reaction chamber is designed to feed its contents to a spectrophotometer 20 or other instrument operable to measure a hematology parameter, such as other commercially available, photometers. To this end, the spectrophotometer 20 is operable to measure the absorption, transmittance, and/or other characteristic of the diluted and lysed test sample. The measured characteristic is then converted into a corresponding measurement for the hematology parameter.

The spectrophotometer includes a light source 21a, a cuvette 21b, and a detector 21c. To arrive at an absorption or transmittance reading, the test sample is passed through the cuvette and the light source emits light through the cuvette and passing test sample. The detector positioned on the opposite side of the cuvette obtains an absorption and/or transmittance reading for the test sample. To convert the absorbance and/or transmittance reading for the test sample into a hematology measurement, a look up table may be used to correlate the reading to the hematology measurement. This is accomplished by a processor 24 and memory 25.

In the embodiment of FIG. 1, the processor 24 and memory 25 are included as part of the automated hematology analyzer. However, the processor and memory may also take a number of different forms, such as a processor in a connected personal computer or other instrument operable to convert the absorbance and/or transmittance reading into an uncorrected hematology measurement, such as haemoglobin concentration. In this embodiment, the processor may be a Pentium IV® or other commercially available microprocessor. The processor 24 in association with the memory 25 is further operable to take the uncorrected hematology measurement and convert it into a corrected hematology parameter, wherein the corrected hematology parameter is based on the uncorrected hematology measurement and the temperature measurement taken by the temperature sensor 17. This corrected hematology measurement compensates for the inaccuracy in the uncorrected hematology measurement due to temperature and provides a more accurate measurement for the hematology parameter measured in the test sample.

The processor 24 is connected to an input/output device 26, such as an LED display screen and a keyboard. In the embodiment of FIG. 1, the I/O device is included on the automated hematology analyzer 10. However, the I/O device may be a peripheral instrument connected to the automated hematology analyzer by an electrical connection, such as a personal computer with a monitor and keyboard. After the diluted and lysed test sample is passed through the cuvette, the sample it is delivered to a disposal chamber 22. The disposal chamber is a relatively large container where spent test samples are deposited for proper waste handling.

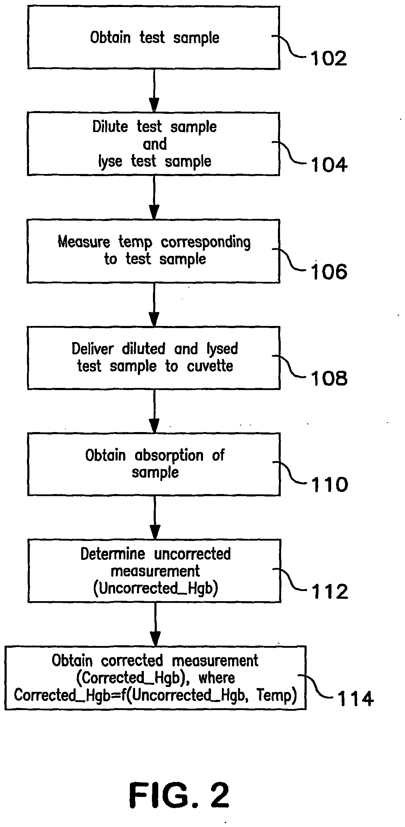

With reference to FIG. 2, operation of the automated hematology analyzer 10 is now described as a series of steps. As shown in step 102, the automated hematology analyzer first obtains a test sample of a patient's blood through the loading platform. After being divided, one aliquot of the test sample is delivered to the reaction chamber and diluted with a diluting agent in step 104. The diluting agent may include a hemolytic reagent added to the diluting agent, or the hemolytic reagent may be added to the sample after it is diluted. In any event, the hemolytic reagent added to the test sample produces a chromogen, and the chromogen allows the spectrophotometer to obtain an absorbance and/or transmittance value for the test sample. Immediately after the reaction of the hemolytic reagent and test sample, a temperature measurement corresponding to the test sample is taken in step 106. In one embodiment, the temperature taken is the temperature of the reaction chamber, which indicates the temperature of the diluted and lysed test sample within the reaction chamber. The measured temperature is delivered to the processor for later use.

Once the test sample is diluted and lysed, it is passed through a cuvette in step 108. As explained above, in one embodiment, the cuvette is part of a spectrophotometer or other measurement instrument. The spectrophotometer obtains an absorption and/or transmittance measurement for the test sample in step 110. This measurement is then passed on to the processor in step 112, where an uncorrected hemoglobin measurement is determined by the processor based on the absorption/transmittance measurement. In one embodiment, the processor determines the uncorrected hemoglobin measurement using look up tables stored in the memory. In particular, to arrive at the uncorrected hemoglobin measurement, the processor simply uses the absorption measurement obtained for the test sample to arrive at a corresponding hemoglobin measurement in the look-up table.

After the uncorrected hemoglobin measurement is determined, the processor obtains a corrected hemoglobin measurement in step 114. The corrected hemoglobin measurement is a function of the uncorrected hemoglobin measurement obtained in step 112, and the temperature measured in step 106.

In order to arrive at an equation for corrected hemoglobin that is a function of the uncorrected hemoglobin measurement and the measured temperature, extensive measurement of hemoglobin variations at different temperatures were recorded for multiple patients. With the hemoglobin measurements for different patients at different temperatures in hand, several mathematical functions were regressed from the data. These functions included both linear and non-linear functions.

One exemplary third order mathematical function that yielded accurate results over a wide temperature range was the following equation (1):

$$Hgb_{Corrected} = Hgb_{Uncorrected} + a_3(T_{Measured}-T_{Ref})^3 + a_2(T_{Measured}-T_{Ref})^2 + a_1(T_{Measured}-T_{Ref}) \quad (1)$$

where,

$Hgb_{Corrected}$ equals the corrected measurement of the hemoglobin concentration,

Hgb$_{Uncorrected}$ equals the uncorrected measurement of the hemoglobin concentration,
T$_{Measured}$ equals the operating temperature,
T$_{Ref}$ equals a reference temperature, and
a$_3$, a$_2$, a$_1$ equal a third, a second, and a first order constants.

In the above equation (1), the reference temperature was 23,89°C (75°F). In addition, the first order constant, second order constant, and third order constant were all determined by the empirical method to regress the equation.

As mentioned previously, the above equation provided an accurate corrected hemoglobin reading over a wide temperature range. However, it is well known that a nonlinear system may be approximated by a linear one given a small enough range. In the present case, it was discovered that a linear function could provide accurate results for measured temperatures between 15,56°C and 32,22°C (60°F and 90°F). In that case, the linear function used to determine the corrected hemoglobin was determined to be as follows:

$$Hgb_{Corrected} = (Cal\_Factor) * [Hgb_{Uncorrected} + (Corr\_Factor_1) * (T_{measured} - T_{standard})] \qquad (2)$$

where
Hgb$_{Corrected}$ is the corrected hemoglobin measurement;
Cal_Factor is a predetermined calibration factor;
Hgb$_{Uncorrected}$ is the uncorrected hemoglobin measurement;
Corr_Factor$_1$ is a predetermined correction factor;
T$_{measured}$ is the measured temperature corresponding to the test sample; and
T$_{standard}$ is a "standard" or reference temperature from which the measured temperature may deviate.

According to the above equation (2), the Cal_Factor, Corr_Factor$_1$, and T$_{standard}$ are all predetermined values (predetermined constants in the equation). To determine the above mathematical function, as well as the predetermined constants, extensive measurement of hemoglobin variations at different temperatures were recorded for multiple patients. With the hemoglobin measurements for different patients at different temperatures in hand, the mathematical function was regressed from the data, and the correction factor (Corr_Factor$_1$), reference temperature (T$_{standard}$) and calibration factor (Cal_Factor) were determined empirically. When determining the calibration factor (Cal_Factor), the temperature reading for normal temperature calibration must factored into the value, as well as the reference temperature and the correction factor, as explained in the following paragraph.

The Cal_Factor value in the above embodiment is determined using a known hemoglobin assay for calibration. Cal_Factor may be dependent upon calibration temperature, the reference temperature, and/or a correction factor. Thus, in one embodiment, the calibration factor is determined according to the following equation (3):

$$Cal\_Factor = Hgb_{known\_assay} / [(Hgb_{measured\_assay}) (T_{calibration}) + Corr\_Factor_2 * (T_{calibration} - T_{standard})] \qquad (3)$$

where
Hgb$_{known\_assay}$ is the known hemoglobin amount for the calibration assay;
Hgb$_{measured\_assay}$ is the measured hemoglobin for the calibration assay;
Corr_Factor$_2$ is a predetermined correction factor;
T$_{calibration}$ is the measured temperature during the calibration; and
T$_{standard}$ is a "standard" or reference temperature from which the calibration temperature may deviate.

The nonlinear and linear equations (1) and (2) provided above are but two representative equations that may be used to arrive at a corrected hemoglobin measurement. As explained above, in both cases a corrected hemoglobin measurement is provided which is a function of an uncorrected hemoglobin measurement and a measured temperature. The above example equations for corrected hemoglobin measurement are not intended as limiting, but are provided as example functions that have been regressed from empirical data. Numerous other functions of uncorrected hemoglobin and measured temperature may be possible to arrive at corrected hemoglobin results.

The following example is illustrative of the method of hemoglobin correction due to temperature correction described herein, and should in no way be interpreted as limiting the invention, as defined in the claims.

EXAMPLE

Hemoglobin concentration was measured using a Beckman Coulter automated hematology analyzer for thirty blood samples. The thirty blood samples were diluted and lysed using Lyse S™ III. Hemoglobin concentration measurements were obtained for each of the thirty blood samples at several different temperatures ranging from 21,11°C to 31,11°C (70°F to 88°F). The temperatures were measured at the reaction chamber of the automated hematology analyzer immediately before the hemoglobin concentration measurements were taken. After uncorrected hemoglobin measurements were obtained, corrected hemoglobin measurements were obtained using the method described herein. Both the uncorrected and corrected hemoglobin results were logged as shown in the tables below. Table 1 below provides the uncorrected hemoglobin concentration measurements for the thirty samples measured. Table 2 below provides the corrected hemoglobin concentration for the thirty samples measured.

| Table 1- Uncorrected Hgb | | | | | |
|---|---|---|---|---|---|
| **SampID** | **Trialyse 70F** | **Trialyse 75F** | **Trialyse 80F** | **Trialyse 85F** | **Trialyse 88F** |
| 1 | 15.05 | 14.89 | 14.57 | 14.47 | 14.23 |
| 2 | 14.56 | 14.3 | 14.02 | 13.85 | 13.68 |
| 3 | 13.69 | 13.5 | 13.21 | 13.17 | 12.92 |
| 4 | 14.58 | 14.22 | 14.01 | 13.84 | 13.67 |
| 5 | 15.07 | 14.91 | 14.61 | 14.49 | 14.31 |
| 6 | 14.84 | 14.72 | 14.38 | 14.15 | 13.99 |
| 7 | 14.83 | 14.53 | 14.41 | 14.21 | 14.01 |
| 8 | 15.3 | 15.19 | 14.86 | 14.6 | 14.48 |
| 9 | 15.13 | 14.88 | 14.75 | 14.52 | 14.34 |
| 10 | 13.72 | 13.48 | 13.29 | 13.17 | 12.96 |
| 11 | 15.13 | 14.85 | 14.43 | 14.51 | 14.27 |
| 12 | 13.74 | 13.55 | 13.39 | 13.21 | 12.97 |
| 13 | 13.17 | 12.88 | 12.79 | 12.64 | 12.4 |
| 14 | 14.03 | 13.82 | 13.71 | 13.47 | 13.26 |
| 15 | 12.76 | 12.53 | 12.38 | 12.26 | 12.01 |
| 16 | 15.49 | 15.33 | 15.08 | 14:85 | 14.74 |
| 17 | 12.3 | 12.15 | 11.93 | 11.82 | 11.45 |
| 18 | 15.25 | 15.06 | 14.84 | 14.68 | 14.51 |
| 19 | 12.44 | 12.43 | 12.15 | 12.04 | 11.9 |
| 20 | 15.27 | 15.07 | 14.79 | 14.6 | 14.4 |
| 21 | 12.45 | 12.24 | 12.01 | 11.85 | 11.68 |
| 22 | 14.5 | 14.3 | 14.2 | 13.99 | 13.87 |
| 23 | 13.94 | 13.72 | 13.53 | 13.34 | 13.08 |
| 24 | 13.53 | 13.32 | 13.1 | 12.92 | 12.8 |
| 25 | 15.11 | 14.98 | 14.78 | 14.49 | 14.37 |
| 26 | 14.67 | 14.52 | 14.3 | 14.06 | 13.87 |
| 27 | 14.46 | 14.28 | 14.12 | 13.94 | 13.75 |
| 28 | 14.06 | 13.9 | 13.67 | 13.5 | 13.32 |
| 29 | 12.37 | 12.14 | 11.97 | 11.67 | 11.58 |
| 30 | 13.66 | 13.56 | 13.38 | 13.07 | 12.95 |

| Table 2- Corrected Hgb | | | | | |
|---|---|---|---|---|---|
| **SampID** | **Trialyse 70F** | **Trialyse 75F** | **Trialyse 80F** | **Trialyse 85F** | **Trialyse 88F** |
| 1 | 14.845 | 14.89 | 14.775 | 14.88 | 14.763 |
| 2 | 14.355 | 14.3 | 14.225 | 14.26 | 14.213 |
| 3 | 13.485 | 13.5 | 13.415 | 13.58 | 13.453 |
| 4 | 14.375 | 14.22 | 14.215 | 14.25 | 14.203 |
| 5 | 14.865 | 14.91 | 14.815 | 14.9 | 14.843 |
| 6 | 14.635 | 14.72 | 14.585 | 14.56 | 14.523 |
| 7 | 14.625 | 14.53 | 14.615 | 14.62 | 14.543 |

(continued)

| | | | Table 2- Corrected Hgb | | |
|---|---|---|---|---|---|
| **SampID** | **Trialyse 70F** | **Trialyse 75F** | **Trialyse 80F** | **Trialyse 85F** | **Trialyse 88F** |
| 8 | 15.095 | 15.19 | 15.065 | 15.01 | 15.013 |
| 9 | 14.925 | 14.88 | 14.955 | 14.93 | 14.873 |
| 10 | 13.515 | 13.48 | 13.495 | 13.58 | 13.493 |
| 11 | 14.925 | 14.85 | 14.635 | 14.92 | 14.803 |
| 12 | 13.535 | 13.55 | 13.595 | 13.62 | 13.503 |
| 13 | 12.965 | 12.88 | 12.995 | 13.05 | 12.933 |
| 14 | 13.825 | 13.82 | 13.915 | 13.88 | 13.793 |
| 15 | 12.555 | 12.53 | 12.585 | 12.67 | 12.543 |
| 16 | 15.285 | 15.33 | 15.285 | 15.26 | 15.273 |
| 17 | 12.095 | 12.15 | 12.135 | 12.23 | 11.983 |
| 18 | 15.045 | 15.06 | 15.045 | 15.09 | 15.043 |
| 19 | 12.235 | 12.43 | 12.355 | 12.45 | 12.433 |
| 20 | 15.065 | 15.07 | 14.995 | 15.01 | 14.933 |
| 21 | 12.245 | 12.24 | 12.215 | 12.26 | 12.213 |
| 22 | 14.295 | 14.3 | 14.405 | 14.4 | 14.403 |
| 23 | 13.735 | 13.72 | 13.735 | 13.75 | 13.613 |
| 24 | 13.325 | 13.32 | 13.305 | 13.33 | 13.333 |
| 25 | 14.905 | 14.98 | 14.985 | 14.9 | 14.903 |
| 26 | 14.465 | 14.52 | 14.505 | 14.47 | 14.403 |
| 27 | 14.255 | 14.28 | 14.325 | 14.35 | 14.283 |
| 28 | 13.855 | 13.9 | 13.875 | 13.91 | 13.853 |
| 29 | 12.165 | 12.14 | 12.175 | 12.08 | 12.113 |
| 30 | 13.455 | 13.56 | 13.585 | 13.48 | 13.483 |

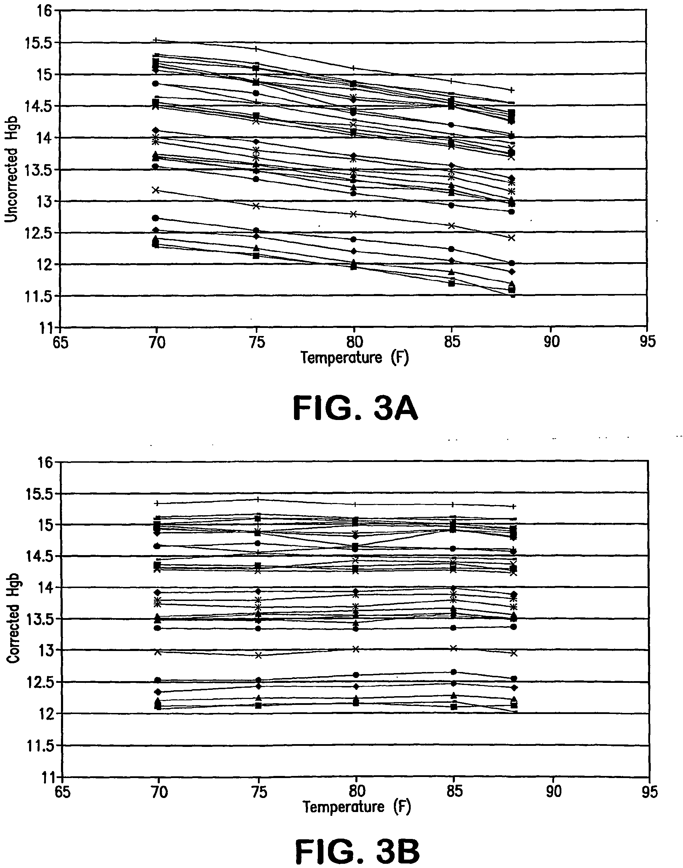

The data from the above tables are provided in graphical form in FIGs. 3A and 3B. FIG. 3A shows the uncorrected hemoglobin concentration results for the thirty blood samples. In FIG. 3A, the horizontal axis represents a temperature measured within the automated hematology analyzer that corresponds to the test sample, in this case, the temperature of the reaction chamber immediately before measurement. The vertical axis represents the measured hemoglobin concentration results. Each line in FIG. 3A shows how the hemoglobin concentrations measured for a single sample vary according to a temperature corresponding to the sample.

FIG. 3B demonstrates the corrected hemoglobin concentration results using the method described herein. Again, the horizontal axis represents the measured temperature. However, the vertical axis represents the corrected hemoglobin concentration obtained as a function of the measured hemoglobin concentration and measured temperature. As can be seen in FIG. 3B, the corrected hemoglobin results are very consistent regardless of the measured temperature.

Although the present invention has been described with respect to certain preferred embodiments, it will be appreciated by those of skill in the art that other implementations and adaptations are possible. For example, different hemolytic reagents could be used, with each producing different equation constants, depending upon the ligands used and the dilution ration.

**Claims**

1.  A method of measuring a hematology parameter of a test sample, the method comprising:

    a) measuring a temperature corresponding to the test sample;
    b) obtaining a first measurement of the hematology parameter of the test sample;
    c) determining a corrected measurement of the hematology parameter of the sample at the measured temperature, wherein the corrected measurement is an empirical function of the measured temperature, a reference temperature, and the first measurement of the hematology parameter, wherein the empirical function is regressed using measurements of hemoglobin variation at different temperatures taken from multiple patients.

2. The method of claim 1 wherein an automated hematology analyzer is used to obtain the first measurement of the hematology parameter of the sample.

3. The method of claim 1 wherein the hematology parameter is a haemoglobin concentration.

4. The method of claim 3 further comprising the step, before step a), of lysing the test sample in a reaction vessel, wherein the step of measuring a temperature corresponding to the test sample comprises measuring a temperature corresponding to the reaction vessel.

5. The method of claim 4 wherein the temperature corresponding to the reaction vessel is a temperature outside of the reaction vessel.

6. The method of claim 4 wherein the temperature corresponding to the reaction vessel is a temperature within the reaction vessel.

7. The method of claim 1 further comprising the step, before step a), of delivering the test sample to a cuvette, wherein the step of measuring a temperature corresponding to the test sample comprises measuring a temperature corresponding to the cuvette.

8. The method of claim 1 wherein the temperature corresponding to the test sample is an ambient air temperature.

9. The method of claim 1 further comprising the step, before step a), of diluting the test sample with a diluting agent, wherein the step of measuring a temperature corresponding to the test sample comprises measuring the temperature corresponding to the diluting agent.

10. The method of claim 1 wherein the function is a linear function valid over a range of test sample temperatures between 15,56°C and 32,22°C (60°F and 90°F).

11. An automated hematology analyzer for measuring a hemoglobin parameter of a test sample, the automated hematology analyzer comprising:

   a) a cuvette operable to receive a test sample;
   b) a light source operable to emit light toward the cuvette;
   c) a detector operable to detect the amount of light from the light source passing through the cuvette and provide a first measurement of the hemoglobin parameter;
   d) a temperature sensor operable to measure a temperature corresponding to the test sample;
   e) a processor operable to determine a corrected measurement of the hemoglobin parameter, wherein the corrected measurement is an empirical function of the first measurement, a reference temperature and the operating temperature, wherein the empirical function is regressed using measurements of hemoglobin variation at different temperatures taken from multiple patients.

12. The automated hematology analyzer of claim 11 wherein the cuvette, light source and detector are provided as part of a spectrophotometer.

13. The automated hematology analyzer of claim 12 wherein the processor is housed within the same housing as the spectrophotometer.

14. The automated hematology analyzer of claim 11 wherein the automated hematology analyzer includes a reaction vessel and the measured temperature is a temperature of the reaction vessel.

15. The automated hematology analyzer of claim 11 wherein the measured temperature is a temperature of the cuvette.

16. The automated hematology analyzer of claim 11 wherein the measured temperature is an ambient air temperature.

17. The automated hematology analyzer of claim 11 wherein the hemoglobin parameter is a hemoglobin concentration.

18. A method of measuring a hemoglobin parameter of a test sample, the method comprising:

a) providing a test sample to an automated hematology analyzer;

b) diluting and lysing the test sample;

c) obtaining a temperature measurement corresponding to the diluted and lysed test sample;

d) obtaining a first measurement of the hemoglobin parameter of the test sample at the measured temperature; and

e) determining a corrected measurement of the hemoglobin parameter, wherein the corrected measurement is an empirical function of the first hemoglobin measurement, a reference temperature and the measured temperature, wherein the empirical function is regressed using measurements of hemoglobin variation at different temperatures taken from multiple patients.

19. The method of claim 18 wherein the corrected measurement is also a function of a standard temperature and at least one correction factor.

20. The method of claim 19 wherein the correction factor is a function of a calibration temperature of the automated hematology analyzer.

21. The method of claim 18 wherein the hemoglobin parameter is hemoglobin concentration.

22. An automated hematology analyzer for measuring a hemoglobin parameter of a sample at an operating temperature, the automated hematology analyzer comprising:

a) means for obtaining a first measurement of the hemoglobin parameter of the sample at the operating temperature;

b) means for measuring the operating temperature; and

c) means for calculating a corrected measurement of the hemoglobin parameter at the operating temperature, wherein the corrected measurement is an empirical function of the first hemoglobin measurement, a reference temperature and the operating temperature, wherein the empirical function is regressed using measurements of hemoglobin variation at different temperatures taken from multiple patients.

23. The automated hematology analyzer of claim 22 wherein the hemoglobin parameter is a hemoglobin concentration.

**Patentansprüche**

1. Verfahren zur Messung eines Hämatologie-Parameters einer Probe, welches Verfahren folgendes aufweist:

a) Messen einer Temperatur, die der Probe entspricht;

b) Erhalten einer ersten Messung des Hämatologie-Parameters der Probe;

c) Bestimmen einer korrigierten Messung des Hämatologie-Parameters der Probe bei der gemessenen Temperatur, wobei die korrigierte Messung eine empirische Funktion der gemessenen Temperatur, eine Referenz-Temperatur und die erste Messung des Hämatologie-Parameters ist, wobei die empirische Funktion durch Regression gewonnen wird, unter Verwendung von Messungen von Hämoglobinvarianten bei verschiedenen Temperaturen, abgenommen von mehreren Patienten.

2. Verfahren nach Anspruch 1, wobei ein automatisierter Hämatologie-Analysator verwendet wird, um die erste Messung des Hämatologie-Parameters der Probe zu erhalten.

3. Verfahren nach Anspruch 1, wobei der Hämatologie-Parameter eine Hämoglobin-Konzentration ist.

4. Verfahren nach Anspruch 3, das des Weiteren, vor Schritt a), den Schritt des Lysierens der Probe in einem Reaktionsgefäß aufweist, wobei der Schritt des Messens einer Temperatur, die der Probe entspricht, den Schritt des Messens einer Temperatur, die dem Reaktionsgefäß entspricht, aufweist.

5. Verfahren nach Anspruch 4, wobei die Temperatur, die dem Reaktionsgefäß entspricht, eine Temperatur außerhalb des Reaktionsgefäßes ist.

6. Verfahren nach Anspruch 4, wobei die Temperatur, die dem Reaktionsgefäß entspricht, eine Temperatur innerhalb des Reaktionsgefäßes ist.

**7.** Verfahren nach Anspruch 1, das des Weiteren, vor Schritt a), den Schritt des Lieferns der Probe an eine Küvette aufweist, wobei der Schritt des Messens einer Temperatur, die der Probe entspricht, das Messen einer Temperatur, die der Küvette entspricht, aufweist.

**8.** Verfahren nach Anspruch 1, wobei die Temperatur, die der Probe entspricht, eine Umgebungsluft-Temperatur ist.

**9.** Verfahren nach Anspruch 1, das des Weiteren, vor Schritt a), den Schritt des Verdünnens der Probe mit einem Verdünnungsmittel aufweist, wobei der Schritt des Messens einer Temperatur, die der Probe entspricht, das Messen der Temperatur, die dem Verdünnungsmittel entspricht, aufweist.

**10.** Verfahren nach Anspruch 1, wobei die Funktion eine lineare Funktion ist, die im Bereich von Proben-Temperaturen zwischen 15,56°C und 32,22°C (60°F und 90°F) gültig ist.

**11.** Automatisierter Hämatologie-Analysator zur Messung eines Hämoglobin-Parameters einer Probe, wobei der Hämatologie-Analysator folgendes aufweist:

> a) eine Küvette, die so operierbar ist, dass sie eine Probe aufnimmt;
> b) eine Lichtquelle, die so operierbar ist, dass sie Licht in Richtung Küvette aussendet;
> c) einen Detektor, der so operierbar ist, dass er die Menge an Licht von der Lichtquelle, das durch die Küvette strömt, erfasst und eine erste Messung des Hämoglobin-Parameters bereitstellt;
> d) einen Temperatursensor, der so operierbar ist, dass er eine Temperatur misst, die der Probe entspricht;
> e) einen Prozessor, der so operierbar ist, dass er eine korrigierte Messung des Hämoglobin-Parameters bestimmt, wobei die korrigierte Messung eine empirische Funktion der ersten Messung, eine Referenztemperatur und die Betriebstemperatur ist, wobei die empirische Funktion durch Regression gewonnen wird, unter Verwendung von Messungen von Hämoglobinvarianten bei verschiedenen Temperaturen, abgenommen von mehreren Patienten.

**12.** Automatisierter Hämatologie-Analysator nach Anspruch 11, wobei die Küvette, die Lichtquelle und der Detektor als Teil eines Spektralfotometers vorgesehen sind.

**13.** Automatisierter Hämatologie-Analysator nach Anspruch 12, wobei der Prozessor innerhalb des selben Gehäuses wie das Spektralfotometer aufgenommen ist.

**14.** Automatisierter Hämatologie-Analysator nach Anspruch 11, wobei der automatisierte Hämatologie-Analysator ein Reaktionsgefäß aufweist und die gemessene Temperatur eine Temperatur des Reaktionsgefäßes ist.

**15.** Automatisierter Hämatologie-Analysator nach Anspruch 11, wobei die gemessene Temperatur eine Temperatur der Küvette ist.

**16.** Automatisierter Hämatologie-Analysator nach Anspruch 11, wobei die gemessene Temperatur eine Umgebungsluft-Temperatur ist.

**17.** Automatisierter Hämatologie-Analysator nach Anspruch 11, wobei der Hämoglobin-Parameter eine Hämoglobin-Konzentration ist.

**18.** Verfahren zur Messung eines Hämoglobin-Parameters einer Probe, welches Verfahren folgendes aufweist:

> a) Bereitstellen einer Probe an einen automatisierten Hämatologie-Analysator;
> b) Verdünnen und Lysieren der Probe;
> c) Erhalten einer Temperaturmessung, die der verdünnten und lysierten Probe entspricht;
> d) Erhalten einer ersten Messung des Hämoglobin-Parameters der Probe bei der gemessenen Temperatur; und
> e) Feststellen einer korrigierten Messung des Hämoglobin-Parameters, wobei die korrigierte Messung eine empirische Funktion der ersten Hämoglobin-Messung, eine Referenztemperatur und die gemessene Temperatur ist, wobei die empirische Funktion durch Regression gewonnen wird, unter Verwendung von Messungen von Hämoglobinvarianten bei verschiedenen Temperaturen, abgenommen von mehreren Patienten.

**19.** Verfahren nach Anspruch 18; wobei die korrigierte Messung auch eine Funktion einer Standard-Temperatur und mindestens eines Korrektur-Faktors ist.

**20.** Verfahren nach Anspruch 19, wobei der Korrektur-Faktor eine Funktion einer Kalibrier-Temperatur des automatisierten Hämoglobin-Analysators ist.

**21.** Verfahren nach Anspruch 18, wobei der Hämoglobin-Parameter eine Hämoglobin-Konzentration ist.

**22.** Automatisierter Hämatologie-Analysator zur Messung eines Hämoglobin-Parameters einer Probe bei einer Betriebstemperatur, wobei der automatisierte Hämatologie-Analysator folgendes aufweist:

> a) Mittel zum Erhalt einer ersten Messung des Hämoglobin-Parameters der Probe bei der Betriebstemperatur;
> b) Mittel zur Messung der Betriebstemperatur; und
> c) Mittel zur Berechnung einer korrigierten Messung des Hämoglobin-Parameters bei der Betriebstemperatur, wobei die korrigierte Messung eine empirische Funktion der ersten Hämoglobin-Messung, eine Referenztemperatur und die Betriebstemperatur ist, wobei die empirische Funktion durch Regression gewonnen wird, unter Verwendung von Messungen von Hämoglobinvarianten bei verschiedenen Temperaturen, abgenommen von mehreren Patienten.

**23.** Automatisierter Hämatologie-Analysator nach Anspruch 22, wobei der Hämoglobin-Parameter eine Hämoglobin-Konzentration ist.

## Revendications

**1.** Procédé destiné à mesurer un paramètre hématologique d'un échantillon pour essai, le procédé comprenant les étapes consistant à :

> a) mesurer une température correspondant à l'échantillon pour essai ;
> b) obtenir une première mesure du paramètre hématologique de l'échantillon pour essai ;
> c) déterminer une mesure corrigée du paramètre hématologique de l'échantillon à la température mesurée, dans lequel la mesure corrigée est une fonction empirique de la température mesurée, d'une température de référence et de la première mesure du paramètre hématologique, dans lequel la fonction empirique est régressée en utilisant des mesures de variation d'hémoglobine à différentes températures prises chez de multiples patients.

**2.** Procédé selon la revendication 1, dans lequel un analyseur d'hématologie automatisé est utilisé pour obtenir la première mesure du paramètre hématologique de l'échantillon.

**3.** Procédé selon la revendication 1, dans lequel le paramètre hématologique est une concentration en hémoglobine.

**4.** Procédé selon la revendication 3, comprenant en outre une étape, antérieure à l'étape a), consistant à lyser l'échantillon pour essai dans un récipient de réaction, dans lequel l'étape consistant à mesurer une température correspondant à l'échantillon pour essai comprend la mesure d'une température correspondant au récipient de réaction.

**5.** Procédé selon la revendication 4, dans lequel la température correspondant au récipient de réaction est une température qui règne à l'extérieur du récipient de réaction.

**6.** Procédé selon la revendication 4, dans lequel la température correspondant au récipient de réaction est une température qui règne à l'intérieur du récipient de réaction.

**7.** Procédé selon la revendication 1, comprenant en outre une étape, antérieure à l'étape a), consistant à placer l'échantillon pour essai dans une cuve, dans lequel l'étape consistant à mesurer une température correspondant à l'échantillon pour essai comprend la mesure d'une température correspondant à la cuve.

**8.** Procédé selon la revendication 1, dans lequel la température correspondant à l'échantillon pour essai est la température de l'air ambiant.

**9.** Procédé selon la revendication 1, comprenant en outre une étape, antérieure à l'étape a), consistant à diluer l'échantillon pour essai avec un diluant, dans lequel l'étape consistant à mesurer une température correspondant à l'échantillon pour essai comprend la mesure de la température correspondant au diluant.

**10.** Procédé selon la revendication 1, dans lequel la fonction est une fonction linéaire valide sur une plage de températures d'échantillon pour essai comprise entre 15,56 °C et 32,22 °C (entre 60 °F et 90 °F).

**11.** Analyseur d'hématologie automatisé destiné à mesurer un paramètre d'hémoglobine d'un échantillon pour essai, l'analyseur d'hématologie automatisé comprenant :

a) une cuve fonctionnelle pour recevoir un échantillon pour essai ;
b) une source de lumière fonctionnelle pour émettre une lumière vers la cuve ;
c) un détecteur fonctionnel pour détecter la quantité de lumière en provenance de la source de lumière passant à travers la cuve et pour fournir une première mesure du paramètre d'hémoglobine ;
d) un capteur de température fonctionnel pour mesurer une température correspondant à l'échantillon pour essai ;
e) un processeur fonctionnel pour déterminer une mesure corrigée du paramètre d'hémoglobine, dans lequel la mesure corrigée est une fonction empirique de la première mesure, d'une température de référence et de la température de fonctionnement ;

dans lequel la fonction empirique est régressée en utilisant des mesures de variation d'hémoglobine à différentes températures prises chez de multiples patients.

**12.** Analyseur d'hématologie automatisé selon la revendication 11, dans lequel la cuve, la source de lumière et le détecteur sont prévus en tant qu'éléments d'un spectrophotomètre.

**13.** Analyseur d'hématologie automatisé selon la revendication 12, dans lequel le processeur est logé à l'intérieur du même logement que le spectrophotomètre.

**14.** Analyseur d'hématologie automatisé selon la revendication 11, dans lequel l'analyseur d'hématologie automatisé comprend un récipient de réaction et la température mesurée est une température du récipient de réaction.

**15.** Analyseur d'hématologie automatisé selon la revendication 11, dans lequel la température mesurée est une température de la cuve.

**16.** Analyseur d'hématologie automatisé selon la revendication 11, dans lequel la température mesurée est la température de l'air ambiant.

**17.** Analyseur d'hématologie automatisé selon la revendication 11, dans lequel le paramètre d'hémoglobine est une concentration en hémoglobine.

**18.** Procédé destiné à mesurer un paramètre d'hémoglobine d'un échantillon pour essai, le procédé comprenant les étapes consistant à :

a) fournir un échantillon pour essai à un analyseur d'hématologie automatisé ;
b) diluer et lyser l'échantillon pour essai ;
c) obtenir une mesure de température correspondant à l'échantillon pour essai dilué et lysé ;
d) obtenir une première mesure du paramètre d'hémoglobine de l'échantillon pour essai à la température mesurée ; et
e) déterminer une mesure corrigée du paramètre d'hémoglobine, dans lequel la mesure corrigée est une fonction empirique de la première mesure d'hémoglobine, d'une température de référence et de la température mesurée, dans lequel la fonction empirique est régressée en utilisant des mesures de variation d'hémoglobine à différentes températures prises chez de multiples patients.

**19.** Procédé selon la revendication 18, dans lequel la mesure corrigée est également une fonction d'une température normale et d'au moins un facteur de correction.

**20.** Procédé selon la revendication 19, dans lequel le facteur de correction est une fonction d'une température d'étalonnage de l'analyseur d'hématologie automatisé.

**21.** Procédé selon la revendication 18, dans lequel le paramètre d'hémoglobine est une concentration en hémoglobine.

**22.** Analyseur d'hématologie automatisé destiné à mesurer un paramètre d'hémoglobine d'un échantillon à une température de fonctionnement, l'analyseur d'hématologie automatisé comprenant :

a) des moyens pour obtenir une première mesure du paramètre d'hémoglobine de l'échantillon à la température de fonctionnement ;
b) des moyens pour mesurer la température de fonctionnement ; et
c) des moyens pour calculer une mesure corrigée du paramètre d'hémoglobine à la température de fonctionnement, dans lequel la mesure corrigée est une fonction empirique de la première mesure d'hémoglobine, d'une température de référence et de la température de fonctionnement, dans lequel la fonction empirique est régressée en utilisant des mesures de variation d'hémoglobine à différentes températures prises chez de multiples patients.

**23.** Analyseur d'hématologie automatisé selon la revendication 22, dans lequel le paramètre d'hémoglobine est une concentration en hémoglobine.

**FIG. 1**

FIG. 2

## FIG. 3A

## FIG. 3B

**EP 1 922 548 B1**

**REFERENCES CITED IN THE DESCRIPTION**

**Patent documents cited in the description**

- US 5763280 A
- US 5968832 A
- WO 2005054811 A